(19) Europäisches Patentamt
European Patent Office
Office européen des brevets

(11) Publication number: 0 209 910 B1

# (12) EUROPEAN PATENT SPECIFICATION

(45) Date of publication of patent specification: 06.12.89

(51) Int. Cl.$^4$: C 07 C 69/675, C 07 C 69/70, C 11 D 1/04

(21) Application number: 86110273.9

(22) Date of filing: 25.07.86

## (54) Surfactants derived from dicarboxylic hydroxyacids.

(30) Priority: 26.07.85 IT 2173485

(43) Date of publication of application:
28.01.87 Bulletin 87/05

(45) Publication of the grant of the patent:
06.12.89 Bulletin 89/49

(84) Designated Contracting States:
BE CH DE FR GB LI NL

(56) References cited:
US-A-3 948 976

(73) Proprietor: AUSCHEM S.p.A.
31, Foro Buonaparte
Milan (IT)

(72) Inventor: Turchini, Luigi
85, via Archimede
I-20129 Milan (IT)
Inventor: Garlisi, Salvatore
32, via Aosta
I-13100 Vercelli (IT)
Inventor: Albanini, Aurelio
37, via Vigorelli
I-27045 Casteggio Pavia (IT)

(74) Representative: Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
D-8000 München 40 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

## Description

The present invention relates to non-toxic, biodegradable surfactants derived from dicarboxylic hydroxyacids and cosmetic and detergent compositions containing them.

In the large number of classes of known surfactants it is uncommon for a single product to combine a variety of characteristics such as biodegradability, non-toxicity, lack of irritating effect on the skin and high solubility in water with excellent detergent properties, making such a product particularly versatile and allowing it to be used in such different areas as, for instance, in detergents in general and, more particularly, for use in cleansing agents, in the food industry, in the textile industry, in emulsion polymerisation, in cosmetics etc.

According to the present invention it has now been found that certain esters which are derived from dicarboxylic hydroxyacids, more particularly, monoesters and/or diesters of tartaric acid or malic acid with polyoxyalkylated aliphatic alcohols as well as the salts of the monoesters with inorganic or organic bases are valuable surfactants. These surfactants exhibit the above-mentioned combination of characteristics and, therefore, are effective in a wide range of applications.

Therefore, the present invention provides cosmetic and detergent compositions containing one or more esters of dicarboxylic hydroxyacids having the general formula:

$$ROOC-\underset{\displaystyle OH}{\underset{|}{CH}}-\underset{\displaystyle R^2}{\underset{|}{CH}}-COOR^1 \qquad (I)$$

wherein:

$R^2$ is H or —OH;

R, $R^1$ are equal to or different from each other and represent a hydrogen atom, a metal ion, an ammonium group, the cation of an organic amine base or a group having the formula:

$$-A_n-R^3 \qquad (II)$$

wherein A is a $C_2$—$C_4$ oxyalkylene group, n is a number of from 1 to 20 and $R^3$ is a $C_8$—$C_{20}$ alkyl group, provided that at least one of said R, $R^1$ is a group —$A_n$—$R^3$. Esters wherein both R and $R^1$ represent the group —A—$R^3$ and wherein the —OH group or —OH groups thereof may optionally be esterified or etherified by conventional methods, are also object of the present invention.

The metal ion is preferably selected from sodium, potassium and magnesium.

The cation of an organic amine base can, for instance, be derived from an alkanolamine, such as monoethanolamine and triethanolamine.

The radical $R^3$ is preferably selected from linear or branched alkyl groups containing from 10 to 16 carbon atoms.

The oxyalkylene group A is preferably an oxyethylene group —$CH_2$—$CH_2$—O— and n preferably has a value of from 4 to 10.

US—A—3 948 976 describes certain esters of hydroxy polycarboxylic acids, some of which correspond to those of the above formula (I). However, said esters are only reported to be useful as additives for inhibiting corrosion and scale on metal parts in a circulating water system while said document does not disclose the valuable surfactant properties thereof.

The esters having the formula (I), may be prepared by esterification of tartaric or malic acid with a polyoxyalkylated aliphatic alcohol having the formula

$$R^3-A_n-OH \qquad (IX)$$

wherein $R^3$, A and n have the meaning specified above, by heating to a temperature of from 150 to 190°C under continuous distillation of the water formed during the reaction and, optionally, final conversion of the obtained product into a salt with metal bases, ammonia or amines.

By the above-mentioned process mono- and diesters having the following formulae are obtained:

monoester of tartaric acid: $$R^3A_nOOC-\underset{\displaystyle OH}{\underset{|}{CH}}-\underset{\displaystyle OH}{\underset{|}{CH}}-COOH \qquad (IV)$$

monoester of malic acid: $$R^3A_nOOC-\underset{\displaystyle OH}{\underset{|}{C}}-CH_2COOH \qquad (V)$$

monoester of malic acid: $$R^3A_nOOC-CH_2-\underset{\displaystyle OH}{\underset{|}{CH}}-COOH \qquad (VI)$$

diester of tartaric acid:

$$R^3A_nOOC—CH—CH—COOA_nR^3 \qquad (VII)$$
$$\quad | \quad |$$
$$OH \quad OH$$

diester of malic acid:

$$R^3A_nOOC—CH—CH_2—COOA_nR^3 \qquad (VIII)$$
$$|$$
$$OH$$

According to the reaction conditions employed and, in particular, depending on the molar ratios of acid to polyoxyalkylated alcohol either mono- or diesters may be prepared, as preferred.

Thus, the acid and the polyoxyalkylated alcohol are reacted in substantially equimolar ratios in order to preferentially produce monoesters having the formulae (IV), (V) and (VI), whereas they are reacted in molar ratios of about 1:2 in order to produce the diesters having formula (VII) or (VIII).

In the case of preparing the monoester of tartaric acid and a polyoxyethylated alcohol, the reaction may be represented by the following equation:

$$HOOC—CH—CH—COOH + R^3\!\!-\!\!(OCH_2CH_2\!\!-\!\!)_nOH \xrightarrow{H_2O} R^3\!\!-\!\!(OCH_2CH_2\!\!-\!\!)_nOOC—CH—CH—COOH$$
$$OH \quad OH \qquad\qquad\qquad\qquad OH \quad OH$$

The polyoxyalkylated alcohols of formula (IV) are known products which are commercially available. Ethoxylated alcohols may be prepared by reaction of the alcohol with ethylene oxide, catalyzed by alkaline bases.

The ethoxylated alcohols having the formula

$$R^3—(OCH_2CH_2)_n—OH,$$

wherein $R^3$ is a linear or branched alkyl group containing from 10 to 16 carbon atoms and n has a value of from 4 to 8, as well as the mixtures thereof are preferred.

The esters employed according to the present invention and, particularly, the monoesters of the ethoxylated aliphatic alcohols, their salts or mixtures thereof, are very efficient surfactants which, although they are used in very low percentages, result in a remarkable lowering of the surface tension and, therefore, may be used as emulsifying agents, dispersants and detergents in general.

They have excellent detergent properties and do not exhibit any noxious or irritating effect on the skin or on the eyes nor do they have any acute toxicity if they are ingested. They are highly biodegradable, their biodegradability being over 90%. They have proved to be stable within a wide temperature range of up to 100°C. They show very good wetting properties and foam-forming ability and their solubility in water is from moderate to excellent. In particular, their solubility increases as the number of moles of ethylene oxide contained in the alkylether chain increases.

The esters employed according to the invention have proved to be compatible with most of the known surfactants and, therefore, they may be combined with them.

Due to their characteristics, the above esters have proved to be very flexible as regards the different applications of surfactants. On account of their high detergent power, combined with the lack of toxic effects on the skin, hair and eyes, they are particularly suitable for cosmetic applications such as, for instance, in the preparation of liquid or creamy detergents for the skin, in shampoos and bathfoams.

The following examples illustrate the invention.

Example 1

Monoesterification of tartaric acid

195.0 g (1.3 moles) of tartaric acid and 660.0 g (1.307 moles) of LIAL® 123.7 ETO (a mixture of ethoxylated alcohols of formula

$$R^3\!\!-\!\!(OCH_2CH_2\!\!-\!\!)_nOH,$$

wherein $R^3=C_{11}$, $C_{12}$, $C_{13}$ and n=7, having an average molecular weight of 505) were introduced, under a nitrogen flow, into a reactor equipped with a heating system, stirrer, thermometer, system for feeding the reactants and connected with a cooler provided with a manifold for collecting the reaction water.

While stirring the mixture under a nitrogen flow, the temperature was raised to 166 to 167°C over a period of about 90 minutes, which is the temperature at which the reaction water begins to distill off and, thereafter, the temperature was increased to 173 to 174°C.

After the distillation of 50% of the theoretical esterification water (corresponding to 23.4 g), the course of the esterification was monitored by determining the acid number.

The esterification is completed after an acid number of 91 to 92 (theoretical=88) and a temperature of

175 to 176°C is reached, which is generally achieved after about 90 minutes of reaction. Thereafter, the reaction mixture was cooled to 80°C and the reactor was discharged.

832 g of a liquid product having a water content lower than 0.1%, an acid number of 89.3 and saponification number of 130.0 were obtained. The analysed product consisted predominantly of the monoester of tartaric acid.

Salification

234.0 g of the ester of tartaric acid thus prepared and 712.0 g of demineralised water were introduced into a vessel equipped with a stirrer, thermometer, dropping funnel and water-cooling system. The mixture was stirred until a solution having a temperature of 20°C was obtained and, thereafter, 54.0 g of a 30% solution of NaOH were introduced slowly, under stirring, over about 1 hour, by means of the dropping funnel, keeping the temperature at values not exceeding 22°C.

1000 g of a clear aqueous solution containing 25% by weight of the sodium salt of the tartaric monoester were obtained.

The solution thus obtained can be used either as such or in a diluted form in the different fields of use of detergents.

By removal of water from the solution, a product having a creamy consistency and predominantly consisting of the sodium salt of the tartaric monoester having an acid number of 1.6 and a saponification number of 65.2, an ester number of 63.6 and a pH at 1% of 6.8 was obtained. The sodium salt dissolves in water at any ratio.

Furthermore, the following determinations were carried out on the salified product.

Surface tension

The surface tension measured at 20°C according to the Du Nuoy method was 34.0 dynes/cm at a concentration of 0.25 g/l and 33.6 dynes/cm at a concentration of 1 g/l.

Wetting power

The wetting power, measured on the product at a concentration of 2 g/l in distilled water was 45 seconds.

Foam-forming ability

This was determined with 200 ml of aqueous solution containing 2 g/l of the product, using a plunger system with a bored disk, 50 strokes. The following results were obtained:

| After minutes | ml of foam |
|---|---|
| 0 | 800 |
| 5 | 780 |
| 10 | 740 |
| 15 | 640 |
| 20 | 520 |

Furthermore, the salified product was highly biodegradable, non-toxic, non-irritating and endowed with very good detergency power.

Example 2

Monoesterification of malic acid

176.6 g (1.317 moles) of malic acid and 668.4 g (1.323 moles) of LIAL® 123.7 ETO (a mixture of ethoxylated alcohols of the formula

$$R^3 \leftarrow OCH_2CH_2 \rightarrow OH,$$

wherein $R^3=C_{11}, C_{12}, C_{13}$ and n=7; $\overline{MW}$=505) were introduced, under nitrogen flow, into a reactor equipped with a heating system, stirrer, thermometer, system for feeding the reactants and connected with a cooler provided with a manifold for collecting the reaction water.

Under stirring and nitrogen flow the temperature was raised to 161 to 162°C over a period of about 90 minutes, at which temperature distillation of reaction water began. Thereafter, the temperature was gradually raised to 180°C until 22.0 g of the reaction water (theor.=23.4 g) were distilled) off. After it had cooled down to 80°C, the reactor was discharged.

821 g of a liquid product with a water content lower than 0.1%, an acid number of 94.4 (theor.=90.3) and a saponification number of 182.5 were obtained. The analysed product predominantly consisted of the monoester of malic acid.

Salification

233.0 g of the malic ester thus prepared and 712.0 g of demineralised water were introduced into a vessel equipped with a stirrer, thermometer, dropping funnel and water-cooling system.

The mixture was stirred until a homogeneous solution was obtained and then, under stirring, 55.0 g of a 30% NaOH solution were added slowly over a period of about 60 minutes by means of a dropping funnel, keeping the temperature at values not exceeding 24°C.

1000 g of a clear aqueous solution containing 25% by weight of the sodium salt of the malic monoester were obtained. The solution thus obtained may be used either as such or in a diluted form in the different fields of use of detergents.

By removal of water from the solution, a product having a creamy consistency and predominantly consisting of the sodium salt of the malic monoester, having an acid number of 3.2, a saponification number of 74.4, an ester number of 71.2 and a pH at 1% of 6.8 was obtained. The salified product dissolves in water in any ratio.

The following determinations were carried out on the product.

Wetting power

The wetting power determined at a concentration of the product of 2 g/l in distilled water was 106 seconds.

Foam-forming ability

This was determined with 200 ml of aqueous solution containing 2 g/l of product, using a plunger system with a bored disk, 50 strokes. The following results were obtained:

| After minutes | ml of foam |
|---|---|
| 0 | 800 |
| 5 | 780 |
| 10 | 750 |
| 15 | 620 |
| 20 | 500 |

Surface tension

The surface tension measured at 20°C according to the Du Nuoy method was 34.6 dynes/cm at the concentration of 0.25 g/l and of 34.1 dynes/l at the concentration of 1 g/l.

The salified product was highly biodegradable, non-toxic, non-irritating and endowed with a very good detergency power.

**Claims**

1. Cosmetic and detergent compositions containing one or more esters of general formula:

$$\begin{array}{l} ROOC-\underset{\large OH}{\underset{|}{C}H}-\underset{\large R^2}{\underset{|}{C}H}-COOR^1 \end{array} \qquad (I)$$

wherein:

$R^2$ is H or —OH;

R, $R^1$ are equal to or different from each other and represent a hydrogen atom, a metal ion, an ammonium group, the cation of an organic amine base or a group of the formula:

$$-A_n-R^3 \qquad (II)$$

wherein A is a $C_2$—$C_4$ oxyalkylene group, n is a number of from 1 to 20 and $R^3$ is a $C_8$—$C_{20}$ alkyl group, with the proviso that at least one of said R, $R^1$ is a group —$A_n$—$R^3$.

2. Use of the esters of general formula (I) as defined in claim 1 as surfactants.

3. Diesters of dicarboxylic hydroxy acids of general formula:

$$R^3-A_n-OOC-\underset{\large OH}{\underset{|}{C}H}-\underset{\large R^2}{\underset{|}{C}H}-COO-A_n-R^3 \qquad (III)$$

wherein

$R^2$ is H or —OH;

A is a $C_2$—$C_4$ oxyalkylene group;

n is a number of from 1 to 20; and

$R^3$ is a $C_8$—$C_{20}$ alkyl group.

4. Diesters according to claim 3, wherein the group —OH or the groups —OH of the diesters of formula (III) have been esterified or etherified according to conventional methods.

5. Diesters according to any one of claims 3 and 4, wherein A is an oxyethylene group $—CH_2—CH_2—O—$, n is a number of from 4 to 10 and $R^3$ is an alkyl group containing 10 to 16 carbon atoms.

**Patentansprüche**

1. Kosmetische und Detergens - Zusammensetzungen, enthaltend einen oder mehrere Ester der allgemeinen Formel

$$ROOC—\underset{OH}{\underset{|}{CH}}—\underset{R^2}{\underset{|}{CH}}—COOR^1 \qquad (I)$$

worin:

$R^2$, H oder —OH ist;

R, $R^1$ gleich oder verschieden voneinander sind und ein Wasserstoffatom, ein Metallion, eine Ammoniumgruppe, das Kation einer organischen Aminbase oder eine Gruppe der Formel

$$—A_n—R^3 \qquad (II)$$

darstellen, worin A eine $C_2—C_4$ - Oxyalkylengruppe ist, n eine Zahl von 1 bis 20 ist und $R^3$ eine $C_8—C_{20}$ - Alkylgruppe ist, mit der Maßgabe, daß wenigstens einer der Reste R, $R^1$ eine Gruppe $—A_n—R^3$ ist.

2. Verwendung der Ester der allgemeinen Formel (I), wie in Anspruch 1 definiert, als Tenside.

3. Diester von Hydroxydicarbonsäuren der allgemeinen Formel:

$$R^3—A_n—OOC—\underset{OH}{\underset{|}{CH}}—\underset{R^2}{\underset{|}{CH}}—COO—A_n—R^3 \qquad (III)$$

worin

$R^2$ H oder —OH ist;

A eine $C_2—C_4$ - Oxyalkylengruppe ist;

n eine Zahl von 1 bis 20 ist; und

$R^3$ eine $C_8—C_{20}$ - Alkylgruppe ist.

4. Diester nach Anspruch 3, worin die —OH-Gruppe oder die —OH-Gruppen der Diester der Formel (III) nach üblichen Methoden in einen Ester oder Ether überführt worden sind.

5. Diester nach irgendeinem der Ansprüche 3 und 4, worin A eine Oxyethylengruppe $—CH_2—CH_2—O—$ ist, n eine Zahl von 4 bis 10 ist und $R^3$ eine Alkylgruppe mit 10 bis 16 Kohlenstoffatomen ist.

**Revendications**

1. Compositions cosmétiques et détergentes contenant un ou plusieurs esters de formule générale:

$$ROOC—\underset{OH}{\underset{|}{CH}}—\underset{R^2}{\underset{|}{CH}}—COOR^1 \qquad (I)$$

dans laquelle:

$R^2$ est un atome d'hydrogène ou un groupe OH;

R et $R^1$, identiques ou différents l'un de l'autre, représentent un atome d'hydrogène, un ion métallique, un groupe ammonium, le cation d'une base aminée organique ou un groupe de formule:

$$—A_n—R^3 \qquad (II)$$

dans laquelle:

A est un groupe oxyalkylène en $C_2$ à $C_4$;

n est un nombre compris entre 1 et 20; et

$R^3$ est un radical alkyle en $C_8$ à $C_{20}$, étant etendu qu'au moins des groupes R ou $R^1$ est un groupe $—A_n—R^3$.

2. Utilisation des esters de formule générale (I) tels que définis dans la revendication 1 en tant qu'agents tensioactifs.

3. Diesters des acides hydroxy - dicarboxyliques de formule générale:

$$R^3—A_n—OOC—\underset{OH}{\underset{|}{CH}}—\underset{R^2}{\underset{|}{CH}}—COO—A_n—R^3 \qquad (III)$$

dans laquelle:

$R^2$ est un atome d'hydrogène ou un groupe OH;

A est un groupe oxyalkylène en $C_2$ à $C_4$;

n est un nombre compris entre 1 et 20; et

$R^3$ est un radical alkyle en $C_8$ à $C_{20}$.

4. Diesters selon la revendication 3, dans lesquels le ou les groupes —OH des diesters de formule (III) ont été estérifiés ou éthérifiés à l'aide de méthodes classiques.

5. Diesters selon l'une quelconque des revendications 3 et 4, dans lesquels A est un radical oxyéthylène $—CH_2—CH_2—O—$, n est un nombre compris entre 4 et 10 et $R^3$ est un radical alkyle comprenant 10 à 16 atomes de carbone.